# NOUVEAU FASCICULE DE BREVET EUROPEEN

(11) **EP 0 770 380 B2**
(45) Date de publication et mention de la décision concernant l'opposition: **03.11.2004**
(45) Mention de la délivrance du brevet: 23.07.1997
(21) Numéro de dépôt: 96401923.6
(22) Date de dépôt: 09.09.1996
(51) Int. Cl.: A61K 7/48

(54) **Composition épaissie en milieu aqueux et procédé d'épaississement d'un milieu aqueux**
Wässrige Verdickungsmittelzusammensetzung und Verfahren zur Verdickung wässriger Systeme
Thickened aqueous composition and thickening process of an aqueous phase

(30) Priorité: 18.09.1995 FR 9510915
(43) Date de publication de la demande: 02.05.1997
(73) Titulaire: L'OREAL, 75008 Paris (FR)
(72) Inventeur: Dupuis, Christine, 75018 Paris (FR); Mondet, Jean, 93600 Aulnay-sous-Bois (FR); Audebert, Roland, 95320 Saint Leu la Foret (FR); Tribet, Christophe, 91700 Villiers sur Orge (FR)
(74) Mandataire: Dossmann, Gérard

(56) Documents cités:
- WO-A-91/11984
- DE-A- 4 329 065
- FR-A- 2 598 611
- Feuille d'information de la Société Croda pour Hydrotriticum QL, QM et QS

## Description

La présente invention a trait à une composition en milieu aqueux comprenant un nouveau système épaississant, ainsi qu'à utilisation de ce nouveau système épaississant dans une composition en milieu aqueux utilisée dans le domaine de la cosmétique.

Il est connu d'utiliser en tant qu'agent épaississant des milieux aqueux, des polymères hydrosolubles ou hydrodispersibles, et notamment des polymères éventuellement réticulés. L'épaississement est alors provoqué par l'enchevêtrement des chaînes de polymères, lesdits polymères ayant de préférence une longueur de chaîne importante et un poids moléculaire élevé.
Il est également connu d'utiliser comme épaississant des milieux aqueux, des polymères hydrophiles comportant des groupements hydrophobes se présentant sous forme de séquences, de greffons et/ou de groupes latéraux répartis de façon aléatoire. Ces polymères permettent d'obtenir un épaississement important du milieu même lorsqu'ils sont utitisés en faible quantité. L'épaississement est généré par la formation d'agrégats entre les groupes hydrophobes du polymère, ces agrégats constituant des points de réticulation physique entre les chaînes macromoléculaires.
Toutefois, on a constaté que la présence de polymères hydrophiles à groupements hydrophobes, même en faible quantité, dans des compositions notamment cosmétiques, pouvait modifier de façon indésirable les propriétés cosmétiques desdites compositions, par exemple les propriétés de toucher ou d'étalement.

D'autre part, il est également connu de préparer des compositions capillaires sous forme de gel comprenant des polymères à motifs hydrophobes associés à des tensioactifs ; le gel est alors formé grâce à la formation de micelles mixtes.
Toutefois, on a constaté que la texture obtenue était souvent cassante ce qui rendait la composition difficilement préhensile ; de plus, la présence d'un excès en tensioactif pouvait conduire à certains inconvénients dans le domaine des compositions non rincées.

Ainsi, il subsiste le besoin d'un système épaississant permettant d'épaissir, voire de gélifier, de manière convenable une composition comprenant un milieu aqueux, sans influer sur les propriétés cosmétiques desdites compositions.

La présente invention a pour but de proposer un tel système épaississant, qui permet de plus d'obtenir un épaississement adéquat en utilisant une très faible quantité de polymère épaississant.

Un objet de la présente invention est donc une composition cosmétique épaissie en milieu aqueux comprenant, en tant qu'agent épaississant, l'association d'un polymère amphiphile qui comporte au moins une chaîne grasse et des motifs hydrophiles, et d'une protéine à groupement hydrophobe dans des proportions de 0,8 à 3% en poids et moins de 5% de tensioactif, la protéine étant présente dans des proportions de 0,005 à 0,2% en poids.

Un autre objet de l'invention est l'utilisation de l'association d'une protéine à groupement hydrophobe et d'un polymère amphiphile qui comporte au moins une chaîne grasse et des motifs hydrophiles en tant qu'agent épaississant, notamment dans une composition cosmétique en milieu acqueux.

Encore un autre objet de l'invention est un procédé pour épaissir une composition cosmétique en milieu aqueux, dans lequel on ajoute à ladite composition une protéine à groupement hydrophobe et un polymère amphiphile qui comporte au moins une chaîne grasse et des motifs hydrophiles.

On a donc constaté qu'une telle association permettait d'obtenir, en milieu aqueux, une forte augmentation de la viscosité pouvant aller jusqu'à la gélification totale du milieu.
Il est donc possible de parvenir au même niveau d'épaississement, voire de gélification, en utilisant des quantités plus faibles de polymère, tout en conservant de bonnes propriétés cosmétiques. Il est à noter que le polymère seul, aux quantités auxquelles il est utilisé en présence de protéine, ne permettrait pas forcément d'obtenir un épaississement du milieu.
L'interaction protéine/polymère permet donc de régler très facilement le degré de viscosité du milieu par simple mélange, dans des proportions ajustables à volonté, de deux solutions fluides, à savoir une solution aqueuse de polymère et une solution aqueuse de protéine.

Un avantage de l'invention réside dans le fait qu'il est possible d'obtenir un épaississement convenable du milieu tout en apportant, en plus, certaines propriétés de soin et/ou de traitement de la peau, en choisissant notamment la protéine de manière adéquate.

Ainsi, il peut être envisagé :
. d'apporter des effets hydratants, lorsque la protéine possède ces effets ;
. d'apporter un effet régénérant de la peau, par apport d'acides aminés et de protéines ;
. d'apporter un effet bactéricide doux, en utilisant certaines protéines telles que le lysozyme ;
. d'apporter des effets antiradicaux libres, en utilisant des protéines telles que les superoxydismutases ; De plus, le réseau polymérique peut avoir un certain effet protecteur pour la protéine, notamment vis-à-vis d'agents dénaturants comme la température ou l'acidité, ce qui est particulièrement intéressant lorsque ladite protéine est un enzyme.

D'autre part, la composition cosmétique, et notamment capillaire, obtenue, s'étale aisément, présente une meilleure préhension, ainsi qu'une bonne élimination au rinçage.

Sans être tenu par cette explication, on peut considérer que, dans le cadre de l'invention, l'augmentation de la viscosité du milieu résulte d'une réticulation physique entre les chaînes de polymère et la protéine, ladite réticulation étant réversible et faisant intervenir des associations ou interactions de type hydrophobe entre, d'une part, les groupes hydrophobes du polymère et d'autre part, les sites hydrophobes de la protéine. Ces interactions de type hydrophobe conduisent alors au réseau de gélification.
Selon le type de protéine utilisé, lesdits sites hydrophobes de la protéine peuvent être situés à la surface de la protéine (protéines globulaires ou fibrillaires par exemple) ou peuvent être répartis dans la chaîne de la protéine (protéines à structure non ordonnée par exemple).

- les polymères susceptibles d'être utilisés dans la présente invention sont des polymères amphiphiles qui comportent au moins une chaîne grasse, donc une partie hydrophobe, et des motifs hydrophiles, donc une partie hydrophile.

La partie hydrophobe peut être en nombre réduit vis-à-vis du reste de la chaîne polymérique, et peut se situer latéralement à la chaîne et être répartie de façon aléatoire (copolymères statistiques) ou répartie sous forme de séquences ou de greffons (copolymères blocs ou copolymères séquencés).
On peut utiliser des polymères solubles dans l'eau, ou hydrodispersibles.
On peut également utiliser, en particulier lorsque le copolymère est réticulé partiellement, des polymères 'gonflables' dans l'eau.

Les polymères peuvent être de toute nature chimique; on peut ainsi choisir des polymères naturels, éventuellement modifiés; des polymères radicalaires notamment vinyliques ou acryliques; des polycondensats; et leurs mélanges.
Ils peuvent être ioniques ou non ioniques, et sont de préférence anioniques ou non ioniques.

On peut en particulier citer, parmi les polymères selon l'invention de type naturels dérivés :
- les éthers de cellulose possédant des substituants hydrophobes, qui peuvent être des groupes alkyls ayant un nombre de carbone supérieur à 8.
   On peut citer l'hydroxyéthylcellulose substituée par des groupes hydrophobes. Parmi les produits commerciaux utilisés dans le domaine cosmétique, on peut citer le Natrosol Plus Grade 330 vendu par la société AQUALON.
- les celluloses cationiques quatemisées modifiées par des groupements comportant au moins une chaîne grasse tels que des groupes alkyle, arylalkyle, alkylaryle ou leurs mélanges où les groupes alkyle sont de préférence en C₈-C₂₂ ;
- les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) vendus par la société AMERCHOL et les produits CRODACEL QM, CRODACEL QL (alkyle en C₁₂) et CRODACEL QS (alkyle en C₁₈) vendus par la société CRODA ;
- les galactomannanes possédant des substituants hydrophobes, et notamment la gomme de guar substituée hydrophobe. Certains de ces dérivés sont notamment décrits dans EP281360.
- des pullulans modifiés par des groupes hydrophobes, en particulier des groupes cholestérol.
- des gélatines modifiées par des groupes hydrophobes, et notamment modifiées par des groupes alkyls en C₆ à C₁₈.
- des muccopolysaccharides tels que ceux constitués de glycosaminoglycane et d'acide hyaluronique.

Parmi les polycondensats utilisables dans le cadre de l'invention, on peut citer les polyuréthannes associatifs qui sont des copolymères séquencés non ioniques comportant dans la chaîne, à la fois des séquences hydrophiles de nature le plus souvent polyoxyéthylène et des séquences hydrophobes qui peuvent être des enchaînements aliphatiques seuls et/ou des enchaînements cycloaliphatiques et/ou aromatiques.

Les copolymères séquencés résultant peuvent être du type tribloc ou multibloc. Les séquences hydrophobes peuvent donc être chaque extrémité de la chaîne (copolymères triblocs à séquence centrale polyoxyéthylène) ou réparties à la fois aux extrémités et dans la chaîne (copolymères multiséquencés). Ils peuvent être également en greffons ou en étoile.
On peut citer les polymères décrits dans l'article de ZEYING MA, J. of Appl. Polymer Sci, vol. 49, 1509-27 (1993).
Parmi les polymères commerciaux, on peut citer les SER-AD FX1100 et SER-AD FX1035 de HÜLS.

Parmi les polymères radicalaires selon l'invention, on peut citer les polymères acryliques anioniques, en dispersion aqueuse, désignés généralement sous le nom de HASE (hydrophobically modified alkalisoluble or swellable emulsion).
Ce sont des copolymères acryliques existant sous forme de dispersions dans l'eau à pH acide et qui peuvent se solubiliser dans l'eau par neutralisation complète des groupes anioniques, c'est-à-dire au-delà de pH 8.
Certaines de ces dispersions peuvent être partiellement réticulées, ce qui implique que la neutralisation complète ne provoque pas la solubilisation complète des particules de polymère, mais engendre un fort gonflement de ces particules, provoquant également la gélification du milieu.
Ces copolymères, non réticulés ou réticulés partiellement, sont généralement des terpolymères entre un monomère porteur de groupe acide carboxylique (acide acrylique, méthacrylique), un monomère relativement insoluble à l'eau du type acrylate ou méthacrylate en C₁ à C₄, tel que l'acrylate d'éthyle, et un troisième monomère porteur d'un groupe hydrophobe, qui peut être rattaché latéralement à la chaîne principale.
Ce groupe hydrophobe peut être un groupe alkyle linéaire ou ramifié et/ou un groupe cycloalkyle et/ou un groupe aryle. Le groupe hydrophobe peut être rattaché à la chaîne principale directement par lintermédiaire d'une liaison éther, ester ou amide, carbamate ou urée. Il peut également être rattaché à la chaîne principale par l'intermédiaire d'une séquence polyoxyéthylénée, elle-même fixée à la chaîne par une liaison éther, ester, amide, carbamate ou urée. Dans ce dernier cas, les groupes latéraux sont généralement de petits greffons à séquence hydrophile et hydrophobe et les propriétés d'épaississement des milieux aqueux sont plus performantes.
De telles dispersions aqueuses de polymère sont notamment décrites dans SHAY, Surface Coatings International, 1993 (11) 446-453, et dans tes brevets US4421902, US4423199 et US4663385 de Rohm et Haas, et US4384096 de Dow Corning.
On peut également citer les Acusol 823 et les Acrysol 25 ou 22 de Rohm & Haas.

Parmi les polymères radicalaires selon l'invention, on peut encore citer:
- les copolymères acide acrylique ou acide méthacrylique avec les N-alkylacrylamides, et en particulier, les copolymères acide acrylique/N-alkylacrylamides ayant un groupe alkyle de C₁ à C₂₀ tels que ceux décrits dans l'article MAGNY et al., Double Liaison, 451, p 52-55 (1993). Ils peuvent être obtenus par copolymérisation directe ou par amidification ultérieure de la chaîne d'acide acrylique. Selon le mode opératoire utilisé, les groupes hydrophobes alkyls peuvent être répartis de façon aléatoire (amidification en solution organique homogène) ou sous forme séquencée (amidification en milieu aqueux où l'amine forme initialement des agrégats de type micellaire).
- d'autres copolymères radicalaires anioniques, tels que des copolymères entre un monomère à groupe acide carboxylique, par exemple acide (méth)acrylique, et des (méth)acrylates esters ou amides porteurs de groupes hydrophobes cycloaliphatiques ou aromatiques, tels que des groupes isobornyle ou adamantyle.
   On peut encore citer des copolymères avec des monomères perfluorés en particulier les copolymères avec le (méth)acrylate de perfluorohexyle : des copolymères entre un monomère porteur d'un groupe acide sulfonique (en particulier acide acrylamido-2-mélhyl-2 propane sulfonique, acide styrène sulfonique) et un alkyle (méth)acrylamide possédant au moins 8 carbones.
- des copolymères acryliques non ioniques, et notamment des copolymères du type acrylamide/N-alkylacrylamide, tels que ceux décrits dans GOODWIN et al., Polymer in Aqueous Media = Performance Through Association, (J.E. Glassed) Adv. Chem. Ser. 223 ; Am. Chem. Soc., Washington DC, p 365 (1989).

On peut encore citer:
- les copolymères d'anhydride maléique et de monomères comportant au moins une chaîne grasse tels que les copolymères N-octadécylvinyléther/anhydride maléique comme le produit GANTREZ AN-8194 vendu par la société ISP ;
- les copolymères de l'acide crotonique et de monomères comportant au moins une chaîne grasse tels que les terpolymères acétate de vinyle/ acide crotonique/ néodécanoate de vinyle comme le produit RESINE 28-2930 vendu par la société NATIONAL STARCH ; ou les terpolymères acétate de vinyle/ acide crotonique/ stéarate d'allyle tels que les produits MEXOMERE PV et PB vendus par la société CHIMEX ;
- les polymères d'acide (méth)acrylique modifiés par des groupes comportant au moins une chaîne grasse ou les copolymères d'acide (méth)acrylique et de monomères comportant au moins une chaîne grasse ; ces monomères sont choisis parmi les monomères hydrophobes à chaîne grasse, les monomères amphiphiles comportant une partie hydrophobe à chaîne grasse et une partie hydrophile ou bien leurs mélanges ; on peut citer à titre d'exemple :

- les copolymères réticulés d'acide acrylique/acrylate d'alkyle en C₁₀-C₃₀ tels que les produits PEMULEN TR 1, PEMULEN TR 2, CARBOPOL 1382, CARBOPOL 1342 et CARBOPOL ETD 2020 vendus par la société GOO-DRICH ;
- les copolymères acide (méth)acrylique/acrylate d'éthyle/acrylate d'alkyle tels que le produit ACUSOL 823 vendu par la société ROHM & HAAS et le produit IMPERON R vendu par la société HOECHST;
- les copolymères réticulés acide acrylique/isodécanoate de vinyle tel que le produit STABYLEN 30 vendu par la société 3V ;
- les terpolymères acide acrylique/vinylpyrrolidone/méthacrylate de lauryle tels que les produits ACRYLIDONE LM, ACP-1184, ACP-1194 vendus par la société ISP ;
- les copolymères acide acrylique/(méth)acrylate de lauryle tels que les produits COATEX SX vendus par la société COATEX ;
- les terpolymères acide (méth)acrylique/acrylate d'alkyle/alkyl polyéthoxylé allyl éther tels que les produits RHEO-VIS -CR, -CR3, -CR2 et -CRX vendus par la société ALLIED COLLOIDS ;
- les terpolymères acide méthacrylique/acrylate d'éthyle/stéaryl polyéthoxylé allyl éther tels que les produits SAL-CARE-SC90 et -SC80 vendus par la société ALLIED COLLOIDS (stéaryl polyéthoxylé à 10 moles d'oxyde d'éthylène noté stéareth-10) ;
- les terpolymères acide méthacrylique/acrylate d'éthyle/acrylate de lauryle polyoxyéthyléné tels que le produit RHEO 2000 vendu par COATEX ;
- les terpolymères acide méthacrylique/acrylate d'éthyle/méthacrylate de stéaryle polyoxyéthyléné tels que les produits ACRYSOL 22, ACRYSOL25 et DW-1206A vendus par la société ROHM & HAAS ;
- les copolymères acide méthacrylique/acrylate d'éthyle/acrylate de nonylphénol polyoxyéthyléné tels que le produit RHEO 3000 vendu par COATEX ;
- les copolymères acide acrylique/monoitaconate de stéaryle polyoxyéthyléné ou les copolymères acide acrylique/monoitaconate de cétyle polyoxyéthyléné tels que les produits 8069-72A et 8069-72B vendus par NATIONAL STARCH ;
- les copolymères acide méthacrylique/acrylate de butyle/monomère hydrophobe comportant une chaîne grasse tels que le produit 8069-146A vendu par NATIONAL STARCH;
- les terpolymères acide acrylique/acrylate d'alkyle en C₁₅/acrylate de polyéthylèneglycol(28 moles d'oxyde d'éthylène) tels que le produit DAPRAL GE 202 vendu par la société AKZO ;
- les sels d'un ester d'acide gras partiel d'un polymère acide acrylique/diméthyléthanolamine tels que le produit DAPRAL GE 202 DMA vendu par la société AKZO ;
- les copolymères acide acrylique/acrylate/monomère amphiphile comportant une chaîne grasse à groupements uréthanne tels que le produit ADDITOL VXW 1312 vendu par HOECHST ;
- les polymères acryliques modifiés par des groupes hydrophobes à chaîne grasse tels que le produit CS-0406 vendu par ROHM & HAAS.

Selon leur nature, les polymères selon invention peuvent être utilisés sous forme de solutions aqueuses ou sous forme de dispersions aqueuses.
On peut indifféremment employer un polymère filmogène ou un polymère non filmogène, voire un mélange de polymère filmogène et non filmogène.
Le polymère selon l'invention est donc un polymère dans lequel le pourcentage et/ou la dimension des groupes hydrophobes sont tels que lesdits groupes hydrophobes sont susceptibles de s'associer, en milieu aqueux, avec d'autres groupes hydrophobes, ceux de la protéine.

Les protéines selon l'invention sont des protéines à groupement hydrophobe; ledit groupement peut être naturellement présent dans la protéine, ou être ajouté.
Elles peuvent être quaternisées ou non quaternisées, ioniques ou non ioniques.
En particulier, on peut citer les protéines ayant en tant que groupement hydrophobe, une chaîne grasse, par exemple une chaîne alkyle à 8-20 atomes de carbone.
On peut en particulier citer, seules ou en mélange :
- les protéines globulaires : ce sont généralement des protéines hydrosolubles, de taille réduite, se présentant en solution sous forme de globules compacts de 3 à quelques dizaines de nanomètres parmi lesquelles on peut nommer :
- les albumines telles que les albumines de sérum (BSA), les albumines extraites du blanc d'oeuf (ovalbumine) ou les albumines extraites du lait (lactalbumine).
- certains enzymes tels que le lysozyme ou les protéases, notamment la papaïne et la trypsine.
- les protéines globulaires d'origine végétale, en particulier les protéines de blé ou de soja.
- les protéines fibrillaires : ce sont des protéines qui peuvent s'organiser en longues fibres, parmi lesquelles on peut citer :

- le collagène et ses dérivés tels que le tropocollagène et la gélatine,
- les protéines de structure des muscles comme l'élastine, ou constituant la structure des membranes telles que la spectrine,
- les polymères de petites protéines tels que le filament d'actine ou la fibrine.
- les protéines à structure non ordonnée, sans structure tertiaire, se présentant en solution sous forme de pelotes statistiques, parmi lesquelles on peut citer les caséines et les mucines extraites des mucus (glycoprotéines).
- les protéines dénaturées pouvant donner des agrégats résultant de la dénaturation d'une solution de lactalbumine, par exemple, ou de solutions de diverses albumines, ou de solutions de caséine.
- les polypeptides synthétiques présentant des séquences hydrophobes, parmi lesquels on peut citer des homopolymères polypeptides tels que le chlorhydrate de polylysine, des copolymères présentant des séquences ou des greffons polypeptides.

Parmi les protéines préférées selon l'invention, on peut citer les glycoprotéines, qui peuvent être associées à des muccopolysaccharides, ou encore la papaïne, le BSA ou le lysozyme qui peuvent être associés à des polymères radicalaires.
On peut encore citer la kératine ou ses dérivés, et les hydrolysats de collagène, de protéines ou de fibroine.

Les protéines selon invention peuvent se présenter sous forme de solutions aqueuses ou éventuellement sous forme de dispersions aqueuses.

L'association selon l'invention peut en particulier être utilisée pour épaissir, voire gélifier, des milieux aqueux de manière à obtenir, par exemple des gels aqueux.
Elle peut éventuellement être utilisée dans le cadre de l'épaississement d'émulsions, en particulier pour des émulsions exemptes de tensioactifs, ou pour l'épaississement de dispersions aqueuses.
On peut ainsi envisager des applications notamment dans les domaines de la cosmétique, de la dermatologie ou de l'hygiène, pour l'épaississement notamment de gels de soin ou de nettoyage de la peau ou des cheveux, de gels coiffants, de gels solaires, de gels de maquillage et de gels bucco-dentaires.
On peut également envisager une application dans le domaine des émulsions et notamment dans les émulsions huile-dans-eau, par exemple dans les crèmes de soin, de nettoyage, de maquillage de la peau ou des cheveux, les crèmes solaires, voire capillaires.

Les quantités de protéine et de polymère à ajouter à un milieu aqueux pourront également être déterminées par l'homme du métier sur base de ses connaissances générales. On peut notamment envisager une composition comprenant 0,1 à 15% en poids de polymère, de préférence 0,2 à 10%.
De préférence, on utilise les protéines en une quantité telle que l'on obtient un rapport pondéral protéine/ polymère compris entre 0,1 et 10.
On peut ainsi obtenir une composition épaissie, ayant une viscosité de 200 à 30 000 cp (mPa.s).

Selon l'application envisagée, la composition peut comprendre les constituants habituels à ce type de composition.
On peut citer tout additif usuellement utilisé dans le domaine considéré, tel que les pigments, les charges et/ou les nacres, des antioxydants, des parfums, des conservateurs, des actifs cosmétiques ou pharmaceutiques, des hydratants, des vitamines, des acides gras essentiels, des filtres solaires, des tensioactifs, les autobronzants (DHA par exemple).
Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

L'invention est illustrée plus en détail dans les exemples suivants.
Les exemples 1 à 3 décrivent la préparation de trois polymères utilisés selon l'invention, par amidification d'un homo-polymère d'acide polyacrylique à l'aide d'une amine hydrophobe, selon la méthode de WANG, ACS Symposium Series, 467, p 218-231 (1991).
Les exemples 4 et 5 décrivent les milieux épaissis obtenus par mélange d'une protéine et d'un polymère.

### Exemple 1 : Préparation du polymère A avant un taux de substitution de 6%

On part d'un acide polyacrylique commercial de poids moléculaire 150 000 vendu par POLYSCIENCES en solution aqueuse. Cette solution est purifiée par dialyse sur membrane SPECTRAPOR 4 (seuil de coupure 12000-14000), puis séchée par lyophilisation.
On détermine la quantité d'eau contenue à chaque fois dans le produit lyophilisé.

On introduit 12,31 g d'acide polyacrylique purifié lyophilisé et contenant 15 % en poids d'eau dans un réacteur avec agitation centrale. On ajoute 340 ml de N-méthylpyrrolidone. On dissout à chaud (50°C) sous agitation pendant 18 heures. Lorsque tout est dissous, on élève la température à 60°C. On ajoute goutte à goutte 2,3 g d'octadécylamine solubilisée dans 40 ml de N-méthylpyrrolidone, pour obtenir un mélange homogène dans le réacteur.
On ajoute ensuite 2,04 g de dicyclohexycarbodiimide dissous dans 40 ml de N-méthylpyrrolidone. On laisse réagir le mélange pendant 24 heures sous agitation, à 60°C.
On refroidit au bain de glace. Il y a formation d'un précipité de dicyclohexylurée. On filtre le milieu sur fritté. Le filtrat est neutralisé par du méthanoate de sodium en solution dans le méthanol, en utilisant 2,2 équivalents vis-à-vis de la quantité théorique.
On agite deux heures à température ambiante. Le polymère neutralisé précipite et est filtré puis séché sous pression réduite.
Le précipité est dissous dans 100 ml d'eau permutée. Il se forme un gel qui est purifié vis-à-vis du solvant N-méthyl pyrrolidone retenu par précipitation lente dans 4 litres de méthanol sous agitation. Le précipité est filtré puis à nouveau purifié avec du méthanol, et est séché sous pression réduite.

On obtient le polymère substitué recherché, avec un rendement 90 % vis-à-vis de la théorie.
Le taux de substitution en groupes octadécylacrylamide est déterminé par analyse élémentaire (rapport N/C) et par RMN du proton en solution diluée (0,1 à 1 %).
Les analyses montrent que le taux de substitution est de 6 % (% molaire en groupes hydrophobes introduits).

### Exemple 2 : Préparation du polymère B avant un taux de substitution de 4 %

On opère selon le même mode opératoire que l'exemple 1, en ajustant les quantités d'octadécylamine pour obtenir un taux de substitution de 4 %, soit 1,2 équivalent de dicyclohexylcarbodiimide et neutralisation par 2,2 équivalents de méthanoate de sodium.
Le rendement est supérieur à 90 %. Les analyses RMN et les analyses élémentaires donnent des taux de substitution respectifs de 3,9 % et 4,2 %.

### Exemple 3 :Préparation du polymère C avant un taux de substitution de 8%

On opère avec le même mode opératoire que l'exemple 1, en adaptant la quantité d'octadécylamine pour obtenir un taux de substitution de 8 % en moles.
Le rendement est supérieur à 90 %. Le taux de substitution, déterminé par analyse élémentaire, est de 8,2 %.

### Exempte 4 : Réalisation des mélanges de polymères/protéines

Les trois polymères A, B et C sont associés, à divers pourcentages, avec trois protéines données : le lysozyme, la BSA et la papaïne.
- Le lysozyme est une protéine globulaire dont le rôle physiologique principal est de catalyser l'hydrolyse des parois cellulaires. Il est considéré comme un bactéricide doux. Sa masse moléculaire est de 14400. En solution, le lysozyme se présente sous la forme d'un ellipsoïde dont les dimensions sont environ de 4,5 x 3 x 3 nm. Son point isoélectrique est de 11. A pH 9, le lysozyme est globalement positif avec une charge moyenne proche de 5,7.
   On utilise du lysozyme de blanc d'oeuf d'une pureté de 90 % (SIGMA Chemicals).
- L'albumine du sérum bovin (BSA) est une protéine globulaire qui a pour fonction physiologique principale de transporter les acides gras d'autres composés hydrophobes dans le plasma. Sa masse moléculaire est de 66700. En solution, la BSA a une forme d'ellipsoïde de dimensions 14 x 4 x 4 nm. Son point isoélectrique est de 4,9. A pH 9, la BSA est globalement négative avec une charge moyenne de :

- 27,6. On utilise une BSA de pureté de 95 à 99 % (SIGMA Chemicals).
- La papaïne est une protéine globulaire et une protéase contenue dans le latex des feuilles de carica papaya. Sa masse moléculaire est de 23400. En solution, elle a pratiquement la forme d'une sphère dont le diamètre est de l'ordre de 4 nm. Son point isoélectrique est de 8,75. A pH 9, elle peut être considérée comme globalement neutre.
   On utilise une papaïne vendue par FLUKA.

On prépare, par mélange, trois solutions-mères :
. une solution aqueuse de protéine (concentration de 1 à 10 % en poids),
. une solution aqueuse du polymère entièrement neutralisée à la soude (concentration de 2 à 3 % en poids), et
. une solution tampon phosphate d'ammonium 100 mM en ion phosphate, pH ajusté à 9 par de l'ammoniaque.
   Avant utilisation, les solutions-mères de protéines et de tampon phosphate sont filtrées (filtre Whatman, porosité 0,2 µm).

Par mélange des solutions-mères, on peut préparer différentes compositions selon l'invention, comprenant des concentrations variables en polymère et en protéine.
Elles contiennent toutes 20 mM d'ions phosphate et 400 ppm de conservateur (NaN₃). Après mélange, les bulles emprisonnées sont chassées par centrifugation, puis les compositions obtenues sont conservées 24 heures à + 4°C avant d'effectuer les mesures.

### Exemple 5 : Etude des compositions épaissies obtenues

La caractérisation des compositions obtenues est faite par détermination du spectre mécanique dans un domaine de fréquence compris entre 0,1 et 100 rad/sec. On utilise pour cela un rhéomètre rotatif FLUIDS SPECTROMETERS : RFS II de RHEOMETRIX.
Les mesures sont faites à 25°C sur une géométrie de type cône/plan (angle du cône 0,04 rad), de diamètre 25 ou 50 mm, sous oscillation (en régime dynamique).
La déformation appliquée est égale à 10 % et se situe dans le domaine linéaire de la réponse à la sollicitation sinusoïdale.

On détermine ainsi, pour chaque fréquence w, le module élastique G' et le module de perte G'' du milieu. Le module G' caractérise le caractère élastique du milieu (aspect comportement d'un solide) alors que le module G'' caractérise son aspect de fluide visqueux (aspect comportement d'un fluide).

Lorsqu'on part d'un milieu initialement fluide ou faiblement épaissi (absence de réticulation physique), G'' est supérieur à G' sur toute la gamme de fréquence balayée. Au fur et à mesure que la viscosité augmente par réticulation physique due à l'association polymère/protéine, G' et G'' augmentent. Mais G' augmente plus rapidement, avec la réticulation, que G'' et devient supérieur à G'' lorsque le milieu est gélifié. Plus la valeur de G' est importante, plus la densité de réticulation est importante dans le réseau formé entre les chaînes macromoléculaires du polymère et la protéine.

Le seuil critique de formation du gel peut être déterminé par différentes méthodes physiques, mais au-delà de ce seuil le module élastique G' devient toujours supérieur à G". La comparaison de G' et de G'', sur la gamme de fréquence balayée sert ici de critère pour quantifier l'importance de gélification.
Pour chaque mélange polymère + protéine, on détermine donc les valeurs de G' et G'' en fonction de la fréquence w.
Puis, on étudie révolution de la tangente de pente Tgδ(w) = G'' (w) / G' (w) (selon Te Nijenhuis, Macromol 1989, 22, 411), pour chaque mélange.
On peut ensuite tracer la courbe de Tgδ en fonction de la concentration en protéines, pour chaque valeur de fréquence w.
Les tracés des courbes aux différentes fréquences se coupent en un même point, que l'on définit comme correspondant au point de gel. On peut ainsi déterminer la concentration en protéines qui provoque la gélification du milieu aqueux.

De plus, lorsque la concentration en protéine augmente au-delà du point de gel, le milieu devient de plus en plus rigide; G' augmente très fortement. Si le pourcentage en protéine se trouve en fort excès par rapport au polymère, on peut observer une saturation des sites d'association portés par le polymère, et les macromolécules sont alors peu à peu déconnectées du réseau formé; G' diminue. Au-delà d'un certain excès en protéine, pour certains mélanges, on constate une précipitation du système notamment quand toutes les charges polymères/protéines sont auto-saturées. On peut ainsi déterminer la concentration en protéines provoquant la précipitation.

Les résultats obtenus sont reportés dans les tableaux ci-dessous :
Tous les mélanges sont à pH 9 et contiennent 20 mM de tampon phosphate d'ammonium et 400 ppm de NaN₃.

| Polymère (%) | Lysozyme (%) au point de gel | Lysozyme (%) à la précipitation |
|---|---|---|
| *Polymère A* | | |
| 0,38 % | 0,01 % | 0,8 % |
| 0,53 % | milieu déjà gélifié | 1,0 % |
| 0,83 % | milieu déjà gélifié | 1,5 % |

| *Polymère B* | | |
|---|---|---|
| 0,58 % | 0,040 % | 0,9 % |
| 0,83 % | 0,035 % | 1,1 % |
| 0,92 % | 0,045 % | 1,1 % |
| 1,25 % | milieu déjà gélifié | 1,3 % |

| *Polymère C* | | |
|---|---|---|
| 0,59 % | < 0,01 % | > 1,0 % |

| Polymère (%) | BSA (%) au point de gel | BSA (%) à la précipitation |
|---|---|---|
| *Polymère A* | | |
| 0,83 % | milieu déjà gélifié | --- |

| *Polymère B* | | |
|---|---|---|
| 0,42 % | 0,035 % | --- |
| 0,92 % | 0,035 % | --- |

| Polymère (%) | Papaïne (%) au point de gel | Papaïne (%) à la précipitation |
|---|---|---|
| *Polymère A* | | |
| 0,83 % | milieu déjà gélifié | 2,5 % |

| *Polymère B* | | |
|---|---|---|
| 0,92 % | 0,13 % | 3,5 % |

On constate donc que, dans la majorité des cas, la solution initiale en polymère n'est pas gélifiée et l'ajout d'une faible quantité en protéine provoque la gélification. Dans certains cas, toutefois, le polymère, seul, conduit à l'obtention d'un milieu déjà gélifié.

De plus, on constate également que, même si la concentration de départ en polymère est telle que le polymère peut déjà gélifier le milieu, l'ajout de protéine augmente fortement la valeur de G', c'est-à-dire la force du gel.
Enfin, pour tous les polymères considérés, à toute concentration en polymère, l'ajout de protéine augmente le module G' jusqu'à une valeur maximum qui correspond à une force de gélification maximum.

On peut donc ainsi régler la force de l'épaississement ou de la gélification en choisissant un rapport polymère/protéine pour une protéine donnée et un polymère donné.
Pour un polymère donné, on peut également choisir la protéine et le rapport protéine/polymère pour obtenir l'effet voulu.

### Exemple 6 : mesure de viscosité

On mesure la viscosité à 25°C d'une composition aqueuse comprenant 1% ou 2% en poids de matière active de polymère et 0,1% ou 0,5% en poids de matière active de protéine.

On obtient les résultats suivants :

| Polymère | Protéine | Viscosité |
|---|---|---|
| 1% Acrysol 22 | --- | 1300 cps |
| | 0,1% Lifidrem KPUN | 1500 cps |
| | 0,1% Lipacide PK | 2260 cps |
| | 0,1% Lexeine QX3000 | 2620 cps |
| | 0,1% Hydrotriticum QS | 1500 cps |
| | 0,1% Promois silk LAQ | 3100 cps |
| | | |
| 2% Acrysol 44 | --- | 520 cps |
| | 0,5% Lifidrem KPUN | 1200 cps |
| | 0,5% Croquat WKP | 2550 cps |
| | 0,5% Croquat K | 1200 cps |

Les composés employés sont les suivants :.
. Acrysol 22 de Rohm et Haas : terpolymère acrylate d'éthyle/acide méthacrylique/méthacrylate de stéaryle oxyéthyléné (55/35/10) en solution aqueuse à 30% en poids
. Lifidrem KPUN de Coletica : undecenoyl kératine de plumes en poudre stérilisée
. Lipacide PK de Seppic : acide palmitoyl kératinique
. Lexeine QX3000 de Inolex : hydrolysat de collagène N-hydroxypropyl cocoyl diméthyl ammonium en solution aqueuse à 30%
. Hydrotriticum QS de Croda : hydrolysat de protéines de blé (PM : 3000) quaternisée en solution aqueuse à 30%
. Promois silk LAQ : hydrolysat de fibroine quaternisée à 30% en solution hydroalcoolique Acrysol 44 de Rohm et Haas : polyuréthanne à terminaison alkyle polyéthoxylé en solution à 35% dans un mélange propylène glycol/eau (60/40)
. Croquat WKP de Croda : hydrolysat de kératine de laine (PM : 1300) quaternisée en solution aqueuse à 30%
. Croquat K de Croda : hydrolysat de kératine (PM : 3000) quaternisée en solution aqueuse à 33%.

On constate donc que l'ajout d'une protéine, quaternisée ou non, à un polymère permet d'améliorer le pouvoir épaississant dudit polymère.
De plus, la composition obtenue présente une viscosité adéquate, tout en comprenant une faible quantité d'épaississant.

## Revendications

1. Utilisation de l'association d'une protéine à groupement hydrophobe et d'un polymère amphiphile qui comporte au moins une chaîne grasse et des motifs hydrophiles en tant qu'agent épaississant, dans une composition cosmétique en milieu aqueux.

2. Utilisation selon la revendication 1, dans laquellela protéine à groupement hydrophobe est choisie parmi, seules ou en mélange, les protéines globulaires telles que les albumines et certains enzymes; les protéines globulaires d'origine végétale; les protéines fibrillaires telles que le collagène et ses dérivés, les protéines de structure des muscles ou membranes, et les polymères de petites protéines; les protéines à structure non ordonnée telles que les caséines et les mucines; les protéines dénaturées; les polypeptides synthétiques présentant des séquences hydrophobes.

3. Utilisation selon l'une des revendications précédentes, dans laquelle la protéine est choisie parmi, seul ou en mélange, les albumines de sérum (BSA), le lysozyme et la papaïne.

4. Utilisation selon l'une des revendications précédentes, dans laquelle la composition aqueuse comprend 0,005% a 0,2% en poids de protéines.

5. Utilisation selon ['une des revendications précédentes, dans laquelle le polymère est hydrosoluble, hydrodispersible ou gonflable dans l'eau et se présente en dispersion ou en solution aqueuse.

6. Utilisation selon l'une des revendications précédentes, dans laquelle le polymère est choisi parmi les polymères naturels, éventuellement modifiés; les polymères radicalaires notamment vinyliques ou acrylique; les polycondensats; et leurs mélanges.

7. Utilisation selon l'une des revendications précédentes, dans laquelle le polymère est choisi parmi les éthers de cellulose possédant des substituants hydrophobes; les celluloses cationiques quaternisées modifiées par des groupements comportant au moins une chaîne grasse; les alkylhydroxyéthylcelluloses quaternisées; les galactomannanes possédant des substituants hydrophobes; les pullulans modifiés par des groupes hydrophobes ; les gélatines modifiées par des groupes hydrophobes ; les muccopolysaccharides ; les polyuréthannes associatifs ; les polymères acryliques anioniques à groupes hydrophobes, éventuellement réticulés; les copolymères acide acrylique ou acide méthacrylique avec les N-alkylacrylamides ; les copolymères entre un monomère à groupe acide carboxylique et des (méth)acrylates esters ou amides porteurs de groupes hydrophobes cycloaliphatiques ou aromatiques; des copolymères avec des monomères perfluorés; des copolymères entre un monomère porteur d'un groupe acide sulfonique et un alkyle (meth)acrylamide possédant au moins 8 carbones; des copolyméres acryliques non ioniques à groupes hydrophobes notamment du type acrylamide/N-alkylacrylamide, les copolymères d'anhydride maléique et de monomères comportant au moins une chaîne grasse ; les copolymères de l'acide crotonique et de monomères comportant au moins une chaîne grasse ; les polymères d'acide (méth)acrylique modifiés par des groupes comportant au moins une chaîne grasse; les copolymères d'acide (méth)acrylique et de monomères comportant au moins une chaîne grasse.

8. Utilisation selon l'une des revendications précédentes dans laquelle la composition aqueuse comprend 0,1 à 15% en poids de polymère, de préférence 0,2 à 10%.

9. Utilisation selon l'une des revendications précédentes, dans laquelle le rapport pondéral protéine/polymère est compris entre 0,1 et 10.

10. Utilisation selon l'une des revendications précédentes, dans laquelle la composition aqueuse comprend moins de 5% en poids de tensioactif.

11. Utilisation selon l'une des revendications précédentes, dans laquelle la composition aqueuse a une viscosité de 200 à 30 000 cp (mPa.s).

12. Utilisation selon l'une des revendications prècédentes, dans laquelle la composition aqueuse se présente sous la forme d'un gel de soin ou de nettoyage de la peau ou des cheveux, d'un gel coiffant, d'un gel solaire, d'un gel de maquillage, d'un gel bucco-dentaire, d'une crème de soin, de nettoyage, de maquillage de la peau ou des cheveux, d'une crème solaire, d'une crème capillaire.

13. Composition cosmétique épaissie en milieu aqueux comprenant en tant qu'agent épaississant l'association d'un polymère amphiphile qui comporte au moins une chaîne grasse et des motifs hydrophiles, et d'une protéine à groupement hydrophobe, **caractérisée par le fait qu'**elle comprend 0,8 à 3% en poids d'agents épaississants et qu'elle comprend moins de 5% en poids de tensioactif, la proteine étant présente dans des proportions de 0,005 à 0,2% en poids.

14. Composition selon la revendication 13 ayant une viscosité de 200 à 30000 cps (mPas).

15. Composition selon la revendication13 ou 14, dans laquelle la protéine à groupement hydrophobe est choisie parmi, seules ou en mélange, les protéines globulaires telles que les albumines et certains enzymes; les protéines globulaires d'origine végétale; les protéines fibrillaires telles que le collagène et ses dérivés, les protéines de structure des muscles ou membranes, et les polymères de petites protéines; les protéines à structure non ordonnés telles que les caséines et les mucines; les protéines dénaturées; les polypeptides synthétiques présentant des sequences hydrophobes.

16. Composition selon l'une des revendications 13 à 15, dans laquelle la protéine est choisie, seule ou en mélange, parmi les albumines de sérum (BSA), le lysozyme et la papaïne.

17. Composition selon l'une des revendications 13 à 17, dans laquelle le polymère est hydrosoluble, hydrodispersible ou gonflable dans l'eau et se présente en dispersion ou en solution aqueuse.

18. Composition selon l'une des revendications 13 à 17, dans laquelle le polymère est choisi parmi les polymères naturels, éventuellement modifiés; les polymères radicalaires notamment vinyliques ou acryliques; les polycondensats, et leurs mélanges.

19. Composition selon l'une des revendications 13 à 18, dans laquelle le polymère est choisi parmi les éthers de cellulose possédant des substituants hydrophobes; les celluloses cationiques quaternisées modifiées par des groupements comportant au moins une chaîne grasse; les alkylhydroxyéthylcelluloses quaternisées; les galactomannanes possédant des substituants hydrophobes; les pullulans modifiés par des groupes hydrophobes; les gélatines modifiées par des groupes hydrophobes; les muccopolysaccharides; les polyuréthannes associatifs; les polymères acryliques anioniques à groupes hydrophobes, éventuellement réticulés; les copolymères acide acrylique ou acide méthacrylique avec les N-alkylacrylamides; les copolymères entre un monomère à groupe acide carboxylique et des (méth)acrylates esters ou amides porteurs de groupes hydrophobes cycloaliphatiques ou aromatique; des copolymères avec des monomères perfluorés; des copolymères entre un monomère porteur d'un groupe acide sulfonique et un alkyle (méth)acrylamide possédant au moins 8 carbones; des copolymères acryliques non ioniques à groupes hydrophobes notamment du type acrylamide/N-alkylacrylamide; les copolymères d'anhydride maléique et de monomères comportant au moins une chaîne grasse; les copolymères de l'acide crotonique et de monomères comportant au moins une chaîne grasse; les polymères d'acide (méth)acrylique modifiés par des groupes comportant au moins une chaîne grasse; les copolymères d'acide (méth)acrylique et de monomères comportant au moins une chaîne grasse.

20. Composition selon l'une des revendications 13 à 19, dans laquelle le rapport pondéral protéine/polymère est compris entre 0,1 et 10.

21. Composition selon l'une des revendications 13 à 20, susceptible d'être utilisée dans les domaines de la cosmétique, et se présentant sous la forme d'un gel de soin ou de nettoyage de la peau ou des cheveux, d'un gel coiffant, d'un gel solaire, d'un gel de maquillage, d'un gel bucco-dentaire, d'une crème de soin, de nettoyage, de maquillage de la peau ou des cheveux, d'une crème solaire, d'une crème capillaire.

## Patentansprüche

1. Verwendung eines Proteins mit hydrophober Gruppe in Kombination mit einem amphiphilen Polymer, das mindestens eine Fettkette und hydrophile Einheiten aufweist, als Verdickungsmittel in einer kosmetischen Zusammensetzung in wässrigem Medium.

2. Verwendung nach Anspruch 1, wobei das Protein mit hydrophober Gruppe unter den folgenden Proteinen oder deren Gemischen ausgewählt ist: den globulären Proteinen, wie Albuminen und einigen Enzymen; globulären Proteinen pflanzlicher Herkunft; fibrillären Proteinen, wie Collagen und seinen Derivaten, Proteinen der Muskelstruktur oder Membranstruktur; Polymeren von kleinen Proteinen; Proteinen ungeordneter Struktur, wie den Caseinen und Mucinen, denaturierten Proteinen und synthetischen Polypeptiden, die hydrophobe Sequenzen aufweisen.

3. Verwendung nach einem der vorhergehenden Ansprüche, wobei das Protein unter den Serumalbuminen (BSA), Lysozym und Papain ausgewählt ist.

4. Verwendung nach einem der vorhergehenden Ansprüche, wobei die wässrige Zusammensetzung 0,2 Gew.-% Proteine enthält.

5. Verwendung nach einem der vorhergehenden Ansprüche, wobei das Polymer wasserlöslich, in Wasser dispergierbar oder in Wasser quellfähig ist und in Dispersion oder wässriger Lösung vorliegt.

6. Verwendung nach einem der vorhergehenden Ansprüche, wobei das Polymer unter den natürlichen gegebenenfalls modifizierten Polymeren; durch radikalische Polymerisation hergestellten Polymeren, insbesondere Vinylpolymeren oder Acrylpolymeren; Polykondensaten und deren Gemischen ausgewählt ist.

7. Verwendung nach einem der vorhergehenden Ansprüche, wobei das Polymer unter den Celluloseestern mit hydrophoben Substituenten; quaternisierten kationischen Celluloseverbindungen, die mit Gruppen modifiziert sind, die mindestens eine Fettkette enthalten; quaternisierten Alkylhydroxyethylcellulosen; Galactomannanen mit hydrophoben Substituenten; den mit hydrophoben Gruppen modifizierten Pullulanen; den mit hydrophoben Gruppen modifizierten Gelatinen; Mucopolysacchariden; assoziativen Polyurethanen; anionischen Acrylpolymeren mit hydrophoben Gruppen, die gegebenenfalls vemetzt sind; Copolymeren von Acrylsäure oder (Meth)acrylsäure und N-Alkylacrylamiden; Copolymeren eines Monomers mit Carbonsäuregruppen und (Meth)acrylatestern oder -amiden, die cycloaliphatische oder aromatische hydrophobe Gruppen tragen; Copolymeren mit perfluorierten Monomeren; Copolymeren eines Monomers, das eine Sulfonsäuregruppe trägt, und einem Alkyl(meth)acrylamid mit mindestens 8 Kohlenstoffatomen; nichtionischen Acrylcopolymeren mit hydrophoben Gruppen, insbesondere vom Typ Acrylamid/N-Alkylacrylamid; Copolymeren von Maleinsäureanhydrid und Monomeren, die mindestens eine Fettkette enthalten; Copolymeren von Crotonsäure und Monomeren mit mindestens einer Fettkette; Polymeren von (Meth)acrylsäure, die mit Gruppen modifiziert sind, die mindestens eine Fettkette enthalten; Copolymeren von (Meth)acrylsäure und Monomeren, die mindestens eine Fettkette enthalten, ausgewählt ist.

8. Verwendung nach einem der vorhergehenden Ansprüche, wobei die wässrige Zusammensetzung 0,1 bis 15 Gew.-% Polymer und vorzugsweise 0,2 bis 10 Gew.-% Polymer enthält.

9. Verwendung nach einem der vorhergehenden Ansprüche, wobei das Gewichtsverhältnis Protein/Polymer im Bereich von 0,1 bis 10 liegt.

10. Verwendung nach einem der vorhergehenden Ansprüche, wobei die wässrige Zusammensetzung weniger als 5 Gew.-% grenzflächenaktiven Stoff enthält.

11. Verwendung nach einem der vorhergehenden Ansprüche, wobei die wässrige Zusammensetzung eine Viskosität von 200 bis 30 000 cP (mPa·s) aufweist.

12. Verwendung nach einem der vorhergehenden Ansprüche, wobei die wässrige Zusammensetzung in Form eines wässrigen Gels zur Pflege oder zur Reinigung der Haut oder der Haare, als Frisiergel, Sonnenschutzgel, Schminkgel, Mund/Zahngel, Creme zur Pflege, zur Reinigung, zum Schminken der Haut oder der Haare, Sonnenschutzcreme oder Creme für die Haarbehandlung vorliegt.

13. Dickflüssige kosmetische Zusammensetzung in wässrigem Medium, die als Verdickungsmittel ein amphiphiles Polymer, das mindestens eine Fettkette und hydrophile Einheiten enthält, in Kombination mit einem Protein mit hydrophober Gruppe enthält, **dadurch gekennzeichnet, dass** sie 0,8 bis 3 Gew.-% Verdickungsmittel enthält und dadurch, dass sie weniger als 5 Gew.-% grenzflächenaktiven Stoff enthält, wobei das Protein in einer Menge von 0,005 bis 0,2 Gew.-% enthalten ist.

14. Zusammensetzung nach Anspruch 13, wobei die Viskosität im Bereich von 200 bis 30 000 cP (mPa·s) liegt.

15. Zusammensetzung nach Anspruch 13 oder 14, wobei das Protein mit hydrophober Gruppe unter den folgenden Proteinen oder deren Gemischen ausgewählt ist: den globulären Proteinen, wie Albuminen und einigen Enzymen; globulären Proteinen pflanzlicher Herkunft; fibrillären Proteinen, wie Collagen und seinen Derivaten, Proteinen der Muskelstruktur oder Membranstruktur; Polymeren von kleinen Proteinen; Proteinen ungeordneter Struktur, wie den Caseinen und Mucinen, denaturierten Proteinen und synthetischen Polypeptiden, die hydrophobe Sequenzen aufweisen.

16. Zusammensetzung nach einem der Ansprüche 13 bis 15, wobei das Protein unter den Serumalbuminen (BSA), Lysoczym und Papain oder deren Gemischen ausgewählt ist.

17. Zusammensetzung nach einem der Ansprüche 13 bis 16, wobei das Polymer wasserlöslich, in Wasser dispergierbar oder in Wasser quellfähig ist und in Dispersion oder in einer wässrigen Lösung vorliegt.

18. Zusammensetzung nach einem der Ansprüche 13, bis 17, wobei das Polymer unter den natürlichen Polymeren, die gegebenenfalls modifiziert sind; den durch radikalische Polymerisation hergestellten Polymeren, insbesondere Vinyl- oder Acrylpolymeren; Polykondensaten; und deren Gemischen ausgewählt ist.

19. Zusammensetzung nach einem der Ansprüche 13 bis 18, wobei das Polymer unter den Celluloseestern mit hydrophoben Substituenten; quaternisierten kationischen Celluloseverbindungen, die mit Gruppen modifiziert sind, die mindestens eine Fettkette enthalten; quaternisierten Alkylhydroxyethylcellulosen; Galactomannanen mit hydrophoben Substituenten; den mit hydrophoben Gruppen modifizierten Pullulanen; den mit hydrophoben Gruppen modifizierten Gelatinen; Mucopolysacchariden; assoziativen Polyurethanen; anionischen Acrylpolymeren mit hydrophoben Gruppen, die gegebenenfalls vernetzt sind; Copolymeren von Acrylsäure oder (Meth)acrylsäure und N-Alkylacrylamiden; Copolymeren eines Monomers mit Carbonsäuregruppen und (Meth)acrylatestern oder -amiden, die cycloaliphatische oder aromatische hydrophobe Gruppen tragen; Copolymeren mit perfluorierten Monomeren; Copolymeren eines Monomers, das eine Sulfonsäuregruppe trägt, und einem Alkyl(meth)acrylamid mit mindestens 8 Kohlenstoffatomen; nichtionischen Acrylcopolymeren mit hydrophoben Gruppen, insbesondere vom Typ Acrylamid/N-Alkylacrylamid; Copolymeren von Maleinsäureanhydrid und Monomeren, die mindestens eine Fettkette enthalten; Copolymeren von Crotonsäure und Monomeren mit mindestens einer Fettkette; Polymeren von (Meth)acrylsäure, die mit Gruppen modifiziert sind, die mindestens eine Fettkette enthalten; Copolymeren von (Meth)acrylsäure und Monomeren, die mindestens eine Fettkette enthalten, ausgewählt ist.

20. Zusammensetzung nach einem der Ansprüche 13 bis 19, wobei das Gewichtsverhältnis Protein/Polymer im Bereich von 0,1 bis 10 liegt.

21. Zusammensetzung nach einem der Ansprüche 13 bis 20, die in der Kosmetik verwendet werden kann und in Form eines Gels zur Pflege oder zur Reinigung der Haut oder der Haare, als Frisiergel, Sonnenschutzgel, Schminkgel, Mund/Zahngel, Creme zur Pflege, zur Reinigung, zum Schminken der Haut oder der Haare, Sonnenschutzcreme oder Creme für das Haar vorliegt.

## Claims

1. Use of the combination of a protein containing a hydrophobic group and an amphiphilic polymer which contains at least one fatty chain and hydrophilic units, as a thickening agent, in a cosmetic composition in aqueous medium.

2. Use according to Claim 1, in which the protein with a hydrophobic group is chosen from, alone or as a mixture, globular proteins such as albumins and certain enzymes; globular proteins of plant origin; fibrous proteins such as collagen and derivatives thereof, proteins of muscle structure or of membrane structure, and polymers of small proteins; proteins with an unordered structure such as caseins and mucins; denatured proteins; synthetic polypeptides having hydrophobic sequences.

3. Use according to either of the preceding claims, in which the protein is chosen from, alone or as a mixture, serum albumins (BSA), lysozyme and papain.

4. Use according to one of the preceding claims, in which the aqueous composition comprises 0.005% to 0.2 % by weight of proteins.

5. Use according to one of the preceding claims, in which the polymer is water-soluble, water-dispersible or swellable in water and is in dispersion or in aqueous solution.

6. Use according to one of the preceding claims, in which the polymer is chosen from natural polymers, which may be modified; radical polymers, in particular vinyl or acrylic radical polymers; polycondensates; and mixtures thereof.

7. Use according to one of the preceding claims, in which the polymer is chosen from cellulose ethers possessing hydrophobic substituents; quaternized cationic celluloses modified with groups containing at least one fatty chain; quaternized alkylhydroxyethylcelluloses; galactomannans possessing hydrophobic substituents; pullulans modified with hydrophobic groups; gelatins modified with hydrophobic groups; mucopolysaccharides; associative polyurethanes; anionic acrylic polymers containing hydrophobic groups, which may be crosslinked; acrylic acid or methacrylic acid copolymers with N-alkylacrylamides; copolymers between a monomer containing a carboxylic acid group and (meth)acrylate esters or amides bearing cycloaliphatic or aromatic hydrophobic groups; copolymers with perfluoro monomers; copolymers between a monomer bearing a sulphonic acid group and an alkyl (meth)acrylamide possessing at least 8 carbons; nonionic acrylic copolymers containing hydrophobic groups, in particular of the acrylamide/N-alkylacrylamide type; copolymers of maleic anhydride and of monomers containing at least one fatty chain; copolymers of crotonic acid and of monomers containing at least one fatty chain; polymers of (meth)acrylic acid modified with groups containing at least one fatty chain; copolymers of (meth)acrylic acid and of monomers containing at least one fatty chain.

8. Use according to one of the preceding claims, in which the aqueous composition comprises 0.1 to 15 % by weight of polymer, preferably 0.2 to 10 %.

9. Use according to one of the preceding claims, in which the protein/polymer weight ratio is between 0.1 and 10.

10. Use according to one of the preceding claims, in which the aqueous composition comprises less than 5 % by weight of surfactant.

11. Use according to one of the preceding claims, in which the aqueous composition has a viscosity of from 200 to 30,000 cp (mPa s).

12. Use according to one of the preceding claims, in which the aqueous composition is in the form of a cleansing or care gel for the skin or the hair, a styling gel, an antisun gel, a make-up gel, a buccodental gel, a care cream, cleansing cream or make-up cream for the skin or the hair, an antisun cream or a hair cream.

13. Thickened cosmetic composition in aqueous medium, comprising as a thickening agent the combination of an amphiphilic polymer which contains at least one fatty chain and hydrophilic units, and of a protein containing a hydrophobic group, **characterized in that** it comprises 0.8 to 3 % by weight of thickening agents and that it comprises less than 5 % by weight of surfactant, the protein being present in proportions of 0.005 to 0.2 % by weight.

14. Composition according to Claim 13, having a viscosity of from 200 to 30,000 cp (mPa s).

15. Composition according to Claim 13 or 14, in which the protein with a hydrophobic group is chosen from, alone or as a mixture, globular proteins such as albumins and certain enzymes; globular proteins of plant origin; fibrous proteins such as collagen and derivatives thereof, proteins of muscle structure or of membrane structure, and polymers of small proteins; proteins with an unordered structure such as caseins and mucins; denatured proteins; synthetic polypeptides having hydrophobic sequences.

16. Composition according to one of Claims 13 to 15, in which the protein is chosen, alone or as a mixture, from serum albumins (BSA), lysozyme and papain.

17. Composition according to one of Claims 13 to 16, in which the polymer is water-soluble, water-dispersible or swellable in water and is in dispersion or in aqueous solution.

18. Composition according to one of Claims 13 to 17, in which the polymer is chosen from natural polymers, which may be modified; radical polymers, in particular vinyl or acrylic radical polymers; polycondensates; and mixtures thereof.

19. Composition according to one of Claims 13 to 18, in which the polymer is chosen from cellulose ethers possessing hydrophobic substituents; quaternized cationic celluloses modified with groups containing at least one fatty chain; quaternized alkylhydroxyethylcelluloses; galactomannans possessing hydrophobic substituents; pullulans modified with hydrophobic groups; gelatins modified with hydrophobic groups; mucopolysaccharides; associative polyurethanes; anionic acrylic polymers containing hydrophobic groups, which may be crosslinked; acrylic acid or methacrylic acid copolymers with N-alkylacrylamides; copolymers between a monomer containing a carboxylic acid group and (meth)acrylate esters or amides bearing cycloaliphatic or aromatic hydrophobic groups; copolymers with perfluoro monomers; copolymers between a monomer bearing a sulphonic acid group and an alkyl (meth)acrylamide possessing at least 8 carbons; nonionic acrylic copolymers containing hydrophobic groups, in particular of the acrylamide/N-alkylacrylamide type; copolymers of maleic anhydride and of monomers containing at least one fatty chain; copolymers of crotonic acid and of monomers containing at least one fatty chain; polymers of (meth)acrylic acid modified with groups containing at least one fatty chain; copolymers of (meth)acrylic acid and of monomers containing at least one fatty chain.

20. Composition according to one of Claims 13 to 19, in which the protein/polymer weight ratio is between 0.1 and 10.

21. Composition according to one of Claims 13 to 20, which can be used in the fields of cosmetics, and is in the form of a cleansing or care gel for the skin or the hair, a styling gel, an antisun gel, a make-up gel, a buccodental gel, a care cream, cleansing cream or make-up cream for the skin or the hair, an antisun cream or a hair cream.
